# NOUVEAU FASCICULE DE BREVET EUROPEEN

(11) **EP 1 353 631 B2**
(45) Date de publication et mention de la décision concernant l'opposition: **17.07.2019**
(45) Mention de la délivrance du brevet: 21.09.2011
(21) Numéro de dépôt: 02710981.8
(22) Date de dépôt: 11.01.2002
(51) Int. Cl.: A61K 8/73, A61Q 5/00

(54) **COMPOSITIONS COSMETIQUES CONTENANT UN FRUCTANE, UN POLYSACCHARIDE ET UN AGENT BENEFIQUE ET LEURS UTILISATIONS**
KOSMETISCHE ZUSAMMENSETZUNGEN DIE EIN FRUCTAN, EIN POLYSACCHARID UND EIN PFLEGEMITTEL ENTHALTEN UND DEREN VERWENDUNG
COSMETIC COMPOSITIONS CONTAINING A FRUCTAN, A POLYSACCHARIDE AND A BENEFICIAL AGENT, AND USES THEREOF

(30) Priorité: 12.01.2001 FR 0100408
(43) Date de publication de la demande: 22.10.2003
(73) Titulaire: L'Oréal, 75008 Paris (FR)
(72) Inventeur: FACK, Géraldine, F-92300 Levallois-Perret (FR); POURILLE-GRETHEN, Chrystel, F-92110 Clichy (FR); RESTLE, Serge, F-95390 Saint-Prix (FR)
(74) Mandataire: L'Oreal
(86) Numéro de dépôt international: PCT/FR2002/000105
(87) Numéro de publication internationale: WO 2002/055034

(56) Documents cités:
- EP-A- 0 792 888
- EP-A- 1 174 118
- EP-A1- 0 502 616
- EP-A1- 0 966 949
- WO-A-98/14482
- WO-A1-96/01849
- WO-A1-97/38673
- DE-A- 10 004 644
- FR-A- 2 795 953
- FR-A- 2 795 954
- FR-A1- 2 810 551
- Eau Aroma-Lactée, base de données Mintel. 05.2000

## Description

La présente invention concerne de nouvelles compositions cosmétiques comprenant dans un milieu cosmétiquement acceptable au moins un fructane, au moins un polysaccharide et au moins un agent bénéfique.

Il est bien connu que des cheveux qui ont été sensibilisés (i.e. abîmés et/ou fragilisés) à des degrés divers sous l'action d'agents atmosphériques ou sous l'action de traitements mécaniques ou chimiques, tels que des colorations, des décolorations et/ou des permanentes, sont souvent difficiles à démêler et à coiffer, et manquent de douceur.

On a déjà proposé pour le traitement des matières kératinique et en particulier des cheveux, des associations de polymères à caractères épaississants. Parmi celles-ci, ont été décrites les associations de polysaccharides tels que l'inuline et de terpolymères acrylique à motif uréthane.

De telles associations présentent cependant des inconvénients tels que des problèmes de rinçabilité, des problèmes de stabilité à pH acide, des difficultés de répartition sur les matières kératiniques ainsi que des propriétés cosmétiques insuffisantes.

La demanderesse a maintenant découvert que l'association d'un fructane avec des polysaccharides et des agents bénéfiques permet de remédier à ces inconvénients.

Ainsi, à la suite d'importantes recherches menées sur la question, il a maintenant été trouvé par la Demanderesse qu'en introduisant un fructane et un polysaccharide dans les compositions en particulier capillaires de l'art antérieur à base d'agents bénéfiques, il est possible de limiter, voire supprimer, les problèmes généralement liés à l'emploi de telles compositions.

De plus, cette association apporte une texture fondante aux compositions cosmétiques, c'est à dire qui disparaît rapidement dans la chevelure. Les cheveux traités avec cette composition ont un toucher doux et sans résidus.

Par ailleurs, les compositions de l'invention appliquées sur la peau notamment sous forme de bain moussant ou de gel douche, apportent une amélioration de la douceur de la peau.

EP-792888 décrit une composition cosmétique comprenant une ester d'inuline ayant des activités détergent et un agent bénéfique. Les formes préférées sont des crèmes nettoyants, les compositions pour le rinçage des cheveux et les lotions pour le corps (voir page 3 lignes 39-40, les exemples 27-29 et les revendications).

EP-1174118 publiée le 23 Janvier 2002, cette demande antérieure divulgue des compositions cosmétiques contenant inuline et /ou un dérives et un agent bénéfique pour les matières kératiniques ; des compositions pour le soin du corps, des compositions écran-solaire ; l'utilisation d'une composition pour le soin du corps et l'utilisation d'un inuline pour la fabrication d'une composition cosmétique (voir page 4 paragraphe 12 et les revendications).

WO-9814482 décrit une composition de shampoing, comprenant des dérivés cationiques de inuline, des tensioactifs et l'acide citrique (voir page 6 lignes 14-19 et l'exemple 4 et revendication 1 et 13).

EP-966949 décrit une composition cosmétique, comprenant un polysaccharide et de terpolymères acrylique. Cette composition permet d'obtenir des compositions pour le lavage, la permanente, le défrisage, la coloration, le soin ou anti-solaire.

Les fructanes sont préparés à partir de produits naturels et présentent donc l'avantage d'être biodégrables. Associés à des agents bénéfiques pour les matières kératiniques, ils permettent d'améliorer les performances des compositions de traitement cosmétique par rapport aux combinaisons d'amidons cationiques et d'agents bénéfiques pour les matières kératiniques de l'art antérieur.

Ainsi, selon la présente invention, il est maintenant proposé de nouvelles compositions cosmétiques, comprenant, dans un milieu cosmétiquement acceptable, au moins un fructane, au moins un polysaccharide différent du fructane et au moins un agent bénéfique.

Les différents objets de l'invention vont maintenant être détaillés. L'ensemble des significations et définitions des composés utilisés dans la présente invention données ci-dessous sont valables pour l'ensemble des objets de invention.

On appelle agent bénéfique pour les matières kératiniques, un agent susceptible notamment de protéger, d'embellir, de conditionner, de traiter et/ou de maintenir en forme les matières kératiniques, et en particulier les cheveux.

Les fructanes ou fructosanes sont des oligosaccharides ou des polysaccharides comprenant un enchaînement d'unités anhydrofructose éventuellement associé à un plusieurs résidus saccharidiques différents du fructose. Les fructanes peuvent être linéaires ou ramifiés. Les fructanes peuvent être des produits obtenus directement à partir d'une source végétale ou microbienne ou bien des produits dont la longueur de chaîne a été modifiée (augmentée ou réduite) par fractionnement, synthèse ou hydrolyse en particulier enzymatique. Les fructanes ont généralement un degré de polymérisation de 2 à environ 1000 et de préférence de 3 à environ 60.

On distingue 3 groupes de fructanes. Le premier groupe correspond à des produits dont les unités fructose sont pour la plupart liées par des liaisons β-2-1.Ce sont des fructanes essentiellement linéaires tes que les inulines.

Le second groupe correspond également à des fructoses linéaires mais les unités fructose sont essentiellement liées par des liaisons β-2-6. Ces produits sont des levanes. Le troisième groupe correspond à des frucanes mixtes, c'est à dire ayant des enchainements β-2-6 et β-2-1. Ce sont des fructanes essentiellement ramifiés tes que les graminanes.

Les fructanes préférés sont les inulines. L'inuline peut être obtenue par exemple à partir de chicorée, de dahlia ou de topinambours.

Le fructane est utilisée de préférence en une quantité comprise entre 0,01 et 20% en poids par rapport au poids total de la composition. Plus préférentiellement, cette quantité est comprise entre 0,05 et 15% en poids par rapport au poids total de la composition et encore plus préférentiellement entre 0,1 et 10% en poids.

Les polysaccharides différents des fructanes est généralement épaississant et notamment choisis les glucanes, les amidons modifiés ou non (tels que ceux issus, par exemple, de céréales comme le blé, le maïs ou le riz, de légumes comme le pois blond, de tubercules comme les pommes de terre ou le manioc), l'amylose, l'amylopectine, le glycogène les dextranes, les celluloses et leurs dérivés (méthylcelluloses, hydroxyalkylcelluloses, éthylhydroxyéthylcelluloses, carboxyméthyl-celluloses), les mannanes, les xylanes, les lignines, les arabanes, les galactanes, les galacturonanes, la chitine, les chitosanes, les glucoronoxylanes, les arabinoxylanes, les xyloglucanes, les glucomannanes, les acides pectiques et les pectines, l'acide alginique et les alginates, les arabinogalactanes, les carraghénines, les agars, les glycosaminoglucanes, les gommes arabiques, les gommes Tragacanthe, les gommes Ghatti, les gommes Karaya, les gommes de caroube, les galactomannanes telles que les gommes de guar et leurs dérivés non ioniques (hydroxypropyl guar) et les gommes de xanthane, et leurs mélanges.

D'une manière générale, les composés de ce type, utilisables dans la présente invention, sont choisis parmi ceux qui sont notamment décrits dans "Encyclopedia of Chemical Technology, Kirk-Othmer, Third Edition, 1982, volume 3, pp. 896-900, et volume 15, pp 439-458", dans "Polymers in Nature, par E. A. MacGREGOR et C. T. GREENWOOD, Editions John Wiley & Sons, Chapter 6, pp 240-328, 1980" et dans l'Industrial Gums - Polysaccharides and their Derivatives, Edité par Roy L. WHISTLER, Second Edition, Edition Academic Press Inc.", le contenu de ces trois ouvrages étant totalement inclus dans la présente demande à titre de référence.

On utilisera de préférence, les pectines, les amidons, les gommes de guar, les celluloses et leurs dérivés.

Le polysaccharide est présent à une concentration comprise entre 0,01 % et 20 % en poids par rapport au poids total de la composition, de préférence entre 0,05 % et 10 % en poids.

Les agents bénéfiques pour les matières kératiniques sont choisis parmi :
(3) les cires animales, végétales ou minérales,
(4) les céramides et les pseudo-céramides,
(12) les tensioactifs cationiques,
(15) les huiles minérales, végétales ou animales,
(17) les esters d'acides gras,
et leurs mélanges.

La composition selon l'invention peut comprendre une ou plusieurs cires animales, végétales ou minérales.

Une cire, au sens de la présente invention, est un composé lipophile, solide à température ambiante (environ 25°C), à changement d'état solide/liquide réversible, ayant une température de fusion supérieure à environ 40°C et pouvant aller jusqu'à 200°C, et présentant à l'état solide une organisation cristalline anisotrope. D'une manière générale, la taille des cristaux de la cire est telle que les cristaux diffractent et/ou diffusent la lumière, conférant à la composition qui les comprend un aspect trouble plus ou moins opaque. En portant la cire à sa température de fusion, il est possible de la rendre miscible aux huiles et de former un mélange homogène microscopiquement, mais en ramenant la température du mélange à la température ambiante, on obtient une recristallisation de la cire dans les huiles du mélange, détectable microscopiquement et macroscopiquement (opalescence).

A titre de cires utilisables dans la présente invention, on peut citer les cires d'origine animale telles que la cire d'abeille, le spermaceti, la cire de lanoline et les dérivés de lanoline ; les cires végétales telles que la cire de Carnauba, de Candellila, d'Ouricury, du Japon, le beurre de cacao ou les cires de fibres de liège ou de canne à sucre ; les cires minérales, par exemple, de paraffine, de vaseline, de lignite ou les cires microcristallines ou les ozokérites, et leurs mélanges.

La composition selon l'invention peut comprendre un ou plusieurs céramides et/ou pseudo-céramides. On peut citer en particulier les céramides des classes I, II, III et V selon la classification de DAWNING, et leurs mélanges.

De tels composés sont par exemple :
- le 2-N-linoléoylamino-octadécane-1,3-diol,
- le 2-N-oléoylamino-octadécane-1,3-diol,
- le 2-N-palmitoylamino-octadécane-1,3-diol,
- le 2-N-stéaroylamino-octadécane-1,3-diol,
- le 2-N-béhénoylamino-octadécane-1,3-diol,
- le 2-N-[2-hydroxy-palmitoyl]-amino-octadécane-1,3-diol,
- le 2-N-stéaroyl amino-octadécane-1,3,4 triol et en particulier la N-stéaroyl phytosphingosine,
- le 2-N-palmitoylamino-hexadécane-1,3-diol ou les mélanges de ces composés.

On peut aussi utiliser des mélanges spécifiques tels que par exemple les mélanges de céramide(s) 2 et de céramide(s) 5 selon la classification de DOWNING.

On peut également utiliser les composés de formule (I) pour lesquels R₁ désigne un radical alkyle saturé ou insaturé dérivé d'acides gras en C₁₂-C₂₂ ; R₂ désigne un radical galactosyle ou sulfogalactosyle ; et R₃ désigne un radical hydrocarboné en C₁₂-C₂₂, saturé ou insaturé et de préférence un groupement -CH=CH-(CH₂)₁₂-CH₃.

A titre d'exemple, on peut citer le produit constitué d'un mélange de glycocéramides, vendu sous la dénomination commerciale GLYCOCER par la société WAITAKI INTERNATIONAL BIOSCIENCES.

On peut également utiliser les composés de formule (I) décrits dans les demandes de brevet EP-A-0227994, EP-A-0 647 617, EP-A-0 736 522 et WO 94/07844.

De tels composés sont par exemple le QUESTAMIDE H (bis-(N-hydroxyéthyl N-cétyl) malonamide) vendu par la société QUEST, le N-(2-hydroxyéthyl)-N-(3-cétyloxy-2-hydroxypropyl)amide d'acide cétylique .

On peut également utiliser le N-docosanoyl N-méthyl-D-glucamine décrit dans la demande de brevet WO94/24097.

La composition selon l'invention peut comprendre un ou plusieurs tensioactifs cationiques bien connus en soi, tels que les sels d'amines grasses primaires, secondaires ou tertiaires, éventuellement polyoxyalkylénées ; les sels d'ammonium quaternaire tels que les chlorures ou les bromures de tétraalkylammonium, d'alkylamidoalkyl-trialkylammo-nium, de trialkylbenzylammonium, de trialkylhydroxyalkylammonium ou d'alkylpyridinium; les dérivés d'imidazoline ; ou les oxydes d'amines à caractère cationique.

La composition selon l'invention peut comprendre une ou plusieurs huiles minérales, végétales ou animales. On peut notamment citer comme huiles d'origine végétale, l'huile d'amande douce, l'huile d'avocat, l'huile de ricin, l'huile d'olive, l'huile de jojoba, l'huile de tournesol, l'huile de germes de blé, l'huile de sésame, l'huile d'arachide, l'huile de pépins de raisin, l'huile de soja, l'huile de colza, l'huile de carthame, l'huile de coprah, l'huile de maïs, l'huile de noisette, le beurre de karité, l'huile de palme, l'huile de noyau d'abricot, l'huile de calophyllum ; comme huile d'origine animale, le perhydrosqualène ; comme huiles d'origine minérale, l'huile de paraffine et l'huile de vaseline ; et leurs mélanges.

La composition selon l'invention peut comprendre un ou plusieurs esters. Comme exemples, on peut notamment citer les esters d'acides gras comme le myristate d'isopropyle, le palmitate d'iso-propyle, le palmitate de 2-éthylhexyle, l'huile de Purcellin (octanoate de stéaryle), l'isononanoate d'isononyle ou d'isostéaryle, le lanolate d'isopropyle, et leurs mélanges.

La composition de traitement des matières kératiniques selon l'invention peut contenir un ou plusieurs des agents bénéfiques pour les matières kératiniques décrits ci-dessus, en une quantité totale allant de 0,001 % à 20 % en poids, de préférence de 0,01 % à 10% en poids et plus particulièrement de 0,1 à 5% en poids par rapport au poids total de la composition.

Les compositions de l'invention contiennent en outre avantageusement au moins un agent tensioactif qui est généralement présent en une quantité comprise entre 0,05% et 50% en poids environ, de préférence entre 0,1% et 40% et encore plus préférentiellement entre 0,55% et 30%, par apport au poids total de la composition.

Cet agent tensioactif peut être choisi parmi les agents tensioactifs anioniques, amphotères, non-ioniques, cationiques ou leurs mélanges.

Les tensioactifs convenant à la mise en oeuvre de la présente invention sont notamment les suivants :

### (1) Tensioactif(s) anionique(s) :

Leur nature ne revêt pas, dans le cadre de la présente invention, de caractère véritablement critique.

Ainsi, à titre d'exemple de tensioactifs anioniques utilisables, seuls ou mélanges, dans le cadre de la présente invention, on peut citer notamment (liste non limitative) les sels (en particulier sels alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, α-oléfine-sulfonates, paraffine-sulfonates ; les alkylsulfosuccinates, les alkyléthersulfosuccinates, les alkylamidesulfosuccinates; les alkylsulfosuccinamates ; les alkylsulfoacétates ; les alkylétherphosphates; les acylsarcosinates ; les acyliséthionates et les N-acyltaurates, le radical alkyle ou acyle de tous ces différents composés comportant de préférence de 8 à 24 atomes de carbone, et le radical aryl désignant de préférence un groupement phényle ou benzyle. Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyl-lactylates dont le radical acyle comporte 8 à 20 atomes de carbone. On peut également utiliser des tensioactifs faiblement anioniques, comme les acides d'alkyl D galactoside uroniques et leurs sels ainsi que les acides alkyl (C₆-C₂₄) éther carboxyliques polyoxyalkylénés, les acides alkyl(C₆-C₂₄)aryl éther carboxyliques polyoxyalkylénés les acides alkyl(C₆-C₂₄) amido éther carboxyliques polyoxyalkylénés et leurs sels, en particulier ceux comportant de 2 à 50 groupements oxyde d'éthylène, et leurs mélanges.

Parmi les tensioactifs anioniques, on préfère utiliser selon l'invention les sels d'alkylsulfates et d'alkyléthersufates et leurs mélanges.

### (II) Tensioactif(s) non ionique(s) :

Les agents tensioactifs non-ioniques sont, eux aussi, des composés bien connus en soi (voir notamment à cet égard "Handbook of Surfactants" par M.R. PORTER, éditions Blackie & Son (Glasgow and London), 1991, pp 116-178) et leur nature ne revêt pas, dans le cadre de la présente invention, de caractère critique. Ainsi, ils peuvent être notamment choisis parmi (liste non limitative) les alcools, les alpha-diols, les alkylphénols ou les acides gras polyéthoxylés, poly-propoxylés ou polyglycérolés, ayant une chaîne grasse comportant par exemple 8 à 18 atomes de carbone, le nombre de groupements oxyde d'éthylène ou oxyde de propylène pouvant aller notamment de 2 à 50 et le nombre de groupements glycérol pouvant aller notamment de 2 à 30. On peut également citer les copolymères d'oxyde d'éthylène et de propylène, les condensats d'oxyde d'éthylène et de propylène sur des alcools gras; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les amines grasses polyéthoxylées ayant de préférence 2 à 30 moles d'oxyde d'éthylène ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl (C₁₀ - C₁₄) amines ou les oxydes de N-acylaminopropylmorpholine. On notera que les alkylpolyglycosides constituent des tensioactifs non-ioniques rentrant particulièrement bien dans le cadre de la présente Invention.

### (iii) Tensioactif(s) amphotère(s):

Les agents tensioactifs amphotères, dont la nature ne revêt pas dans le cadre de la présente invention de caractère critique, peuvent être notamment (liste non limitative) des dérivés d'amines secondaires ou tertiaires aliphatiques, dans lesquels le radical aliphatique est une chaîne linéaire ou ramifiée comportant 8 à 22 atomes de carbone et contenant au moins un groupe anionique hydrosolubilisant (par exemple carboxylate, sulfonate, sulfate, phosphate ou phosphonate) ; on peut citer encore les alkyl (C₈-C₂₀) bétaïnes, les sulfobétaïnes, les alkyl (C₈-C₂₀) amidoalkyl (C₁-C₆) bétaïnes ou les alkyl (C₈-C₂₀) amidoalkyl (C₁-C₆) sulfobétaïnes.

Parmi les dérivés d'amines, on peut citer les produits commercialisés sous les dénomination M IRANOL, tels que décrits dans les brevets US-2 528 378 et US-2 781 354 et de structures :

R₂ -CONHCH₂CH₂ -N(R₃)(R₄)(CH₂COO-) (2)

dans laquelle : R₂ désigne un radical alkyle dérivé d'un acide R₂-COOH présent dans l'huile de coprah hydrolysée, un radical heptyle, nonyle ou undécyle, R₃ désigne un groupement bêta-hydroxyéthyle et R₄ un groupement carboxyméthyle ;
et

R₅-CONHCH₂CH₂-N(B)(C) (3)

dans laquelle :
B représente -CH₂CH₂OX', C représente -(CH₂)_{z} -Y', avec z = 1 ou 2,
X' désigne le groupement -CH₂CH₂-COOH ou un atome d'hydrogène
Y' désigne -COOH ou le radical -CH₂- CHOH - SO3H
R₅ désigne un radical alkyle d'un acide R₉ -COOH présent dans l'huile de coprah ou dans l'huile de lin hydrolysée, un radical alkyle, notamment en C₇, C₉, C₁₁ ou C₁₃, un radical alkyle en C₁₇ et sa forme iso, un radical C₁₇ insaturé.

Ces composés sont classés dans le dictionnaire CTFA, 5ème édition, 1993, sous les dénominations Disodium Cocoamphodiacetate, Disodium Lauroamphodiacetate, Disodium Caprylamphodiacetate, Disodium Capryloamphodiacetate, Disodium Coco-amphodipropionate, Disodium Lauroamphodipropionate, Disodium Caprylamphodipropionate, Disodium Capryloamphodipropionate, Lauroamphodipropionic acid, Coco-amphodipropionic acid.

A titre d'exemple on peut citer le cocoamphodiacetate commercialisé sous la dénomination commerciale MIRANOL C2M concentré par la société RHONE POULENC.

### (iv) Les tensioactifs cationiques peuvent être choisis parmi :

A) les sels d'ammonium quaternaires de la formule générale (IV) suivante : 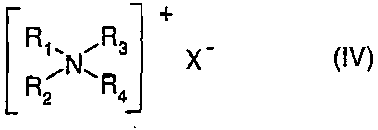 dans laquelle X est un anion choisi dans le groupe des halogénures (chlorure, bromure ou iodure) ou alkyl(C₂-C₆) sulfates plus particulièrement méthylsulfate, des phosphates, des alkyl-ou-alkylarylsulfonates, des anions dérivés d'acide organique tel que l'acétate ou le lactate.
   , et
   a) les radicaux R1 à R3, qui peuvent être identiques ou différents, représentent un radical aliphatique, linéaire ou ramifié, comportant de 1 à 4 atomes de carbone, ou un radical aromatique tel que aryle ou alkylaryle. Les radicaux aliphatiques peuvent comporter des hétéroatomes tels que notamment l'oxygène, l'azote, le soufre, les halogènes. Les radicaux aliphatiques sont par exemple choisis parmi les radicaux alkyle, alcoxy, alkylamide, R4 désigne un radical alkyle, linéaire ou ramifié, comportant de 16 à 30 atomes de carbone.
      De préférence le tensioactif cationique est un sel (par exemple chlorure) de béhényl triméthyl ammonium.
   b) les radicaux R1 et R2, qui peuvent être identiques ou différents, représentent un radical aliphatique, linéaire ou ramifié, comportant de 1 à 4 atomes de carbone, ou un radical aromatique tel que aryle ou alkylaryle. Les radicaux aliphatiques peuvent comporter des hétéroatomes tels que notamment l'oxygène, l'azote, le soufre, les halogènes. Les radicaux aliphatiques sont par exemple choisis parmi les radicaux alkyle, alcoxy, alkylamide et hydroxyalkyle, comportant environ de 1 à 4 atomes de carbone; R3 et R4, identiques ou différents, désignent un radical alkyle, linéaire ou ramifié, comportant de 12 à 30 atomes de carbone, ledit radical comprenant au moins une fonction ester ou amide. R3 et R4 sont notamment choisis parmi les radicaux alkyl(C₁₂-C₂₂)amido alkyle(C₂-C₆), alkyl(C₁₂-C₂₂)acétate ;
      De préférence le tensioactif cationique est un sel (par exemple chlorure) de stéaramidopropyl diméthyl (myristylacétate) ammonium.
B) - les sels d'ammonium quaternaire de l'imidazolinium, comme par exemple celui de formule (V) suivante : 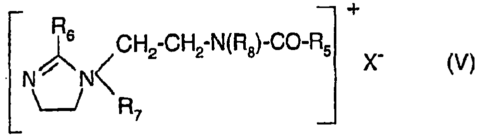 dans laquelle R₅ représente un radical alcényle ou alkyle comportant de 8 à 30 atomes de carbone par exemple dérivés des acides gras du suif, R₆ représente un atome d'hydrogène, un radical alkyle en C₁-C₄ ou un radical alcényle ou alkyle comportant de 8 à 30 atomes de carbone, R₇ représente un radical alkyle en C₁-C₄, R₈ représente un atome d'hydrogène, un radical alkyle en C₁-C₄. X est un anion choisi dans le groupe des halogénures, phosphates, acétates, lactates, alkylsulfates, alkyl-ou-alkylarylsulfonates. De préférence, R₅ et R₆ désignent un mélange de radicaux alcényle ou alkyle comportant de 12 à 21 atomes de carbone par exemple dérivés des acides gras du suif, R₇ désigne méthyle, R₈ désigne hydrogène. Un tel produit est par exemple le Quaternium-27(CTFA 1997) ou le Quaternium-83 (CTFA 1997) commercialisés sous les dénominations "REWOQUAT" W 75, W90, W75PG, W75HPG par la société WITCO,
C) - les sels de diammonium quaternaire de formule (VI) : 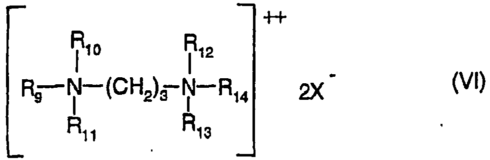 dans laquelle R₉ désigne un radical aliphatique comportant environ de 16 à 30 atomes de carbone, R₁₀, R₁₁, R₁₂, R₁₃ et R₁₄, identiques ou différents sont choisis parmi l'hydrogène ou un radical alkyle comportant de 1 à 4 atomes de carbone, et X est un anion choisi dans le groupe des halogénures, acétates, phosphates, nitrates et méthylsulfates. De tels sels de d'ammonium quaternaire comprennent notamment le dichlorure de propanesuif diammonium.
D) - les sels d'ammonium quaternaire contenant au moins une fonction ester de formule (VII) suivante : 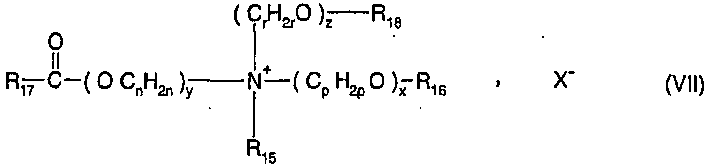 dans laquelle :
   - R₁₅ est choisi parmi les radicaux alkyles en C₁-C₆ et les radicaux hydroxyalkyles ou dihydroxyalkyles en C₁-C₆ ;
   - R₁₆ est choisi parmi :
   - le radical 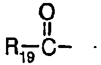
   - les radicaux R₂₀ hydrocarbonés en C₁-C₂₂ linéaires ou ramifiés, saturés ou insaturés,
   - l'atome d'hydrogène,
   - R₁₈ est choisi parmi :
   - le radical 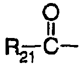
   - les radicaux R₂₂ hydrocarbonés en C₁-C₆ linéaires ou ramifiés, saturés ou insaturés,
   - l'atome d'hydrogène,
   - R₁₇, R₁₉ et R₂₁, identiques ou différents, sont choisis parmi les radicaux hydrocarbonés en C₇-C₂₁, linéaires ou ramifiés, saturés ou insaturés ;
   - n, p et r, identiques ou différents, sont des entiers valant de 2 à 6 ;
   - y est un entier valant de 1 à 10 ;
   - x et z, identiques ou différents, sont des entiers valant de 0 à 10 ;
   - X⁻ est un anion simple ou complexe, organique ou inorganique ;
   sous réserve que la somme x + y + z vaut de 1 à 15 , que lorsque x vaut 0 alors R₁₆ désigne R₂₀ et que lorsque z vaut 0 alors R₁₈ désigne R₂₂.

On utilise plus particulièrement les sels d'ammonium de formule (VII) dans laquelle :
- R₁₅ désigne un radical méthyle ou éthyle,
- x et y sont égaux à 1 ;
- z est égal à 0 ou 1 ;
- n, p et r sont égaux à 2 ;
- R₁₆ est choisi parmi :
- le radical 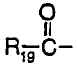
- les radicaux méthyle, éthyle ou hydrocarbonés en C₁₄-C₂₂
- l'atome d'hydrogène ;
- R₁₇, R₁₉ et R₂₁, identiques ou différents, sont choisis parmi les radicaux hydrocarbonés en C₇-C₂₁, linéaires ou ramifiés, saturés ou insaturés ;
- R₁₈ est choisi parmi:
- le radical 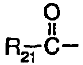
- l'atome d'hydrogène ;

De tels composés sont par exemple commercialisés sous les dénominations DEHYQUART par la société HENKEL, STEPANQUAT par la société STEPAN, NOXAMIUM par la société CECA, REWOQUAT WE 18 par la société REWO-WITCO.

Parmi les sels d'ammonium quaternaire on préfère le chlorure de béhényltriméthylammonium, ou encore, le chlorure de stéaramidopropyldiméthyl (myristyl acétate) ammonium commercialisé sous la dénomination "CERAPHYL 70» par la société VAN DYK, le Quatemium-27 ou le Quaternium-83 commercialisés par la société WITCO.

Dans les compositions conformes à l'invention, on peut utiliser des mélanges d'agents tensioactifs et en particulier des mélanges d'agents tensioactifs anioniques, des mélanges d'agents tensioactifs anioniques et d'agents tensioactifs amphotères, cationiques ou non ioniques, des mélanges d'agents tensioactifs cationiques avec des agents tensioactifs non ioniques ou amphotères. Un mélange particulièrement préféré est un mélange constitué d'au moins un agent tensioactif anionique et d'au moins un agent tensioactif amphotère.

La composition de l'invention peut également contenir au moins un additif choisi parmi les épaississants polymériques naturels ou synthétiques, anioniques, amphotères, zwitteroniques, non ioniques ou cationiques, associatifs ou non, des épaississants non polymériques comme des acides ou des électrolytes, les parfums, les agents nacrants, les conservateurs, les colorants, les agents de pH, les particules minérales ou organiques, les vitamines, les provitamines, le panthénol, et tout autre additif classiquement utilisé dans le domaine cosmétique qui n'affecte pas les propriétés des compositions selon l'invention.

Ces additifs sont présents dans la composition selon l'invention dans des proportions pouvant aller de 0 à 40% en poids par rapport au poids total de la composition. La quantité précise de chaque additif est déterminée facilement par l'homme du métier selon sa nature et sa fonction.

Les compositions selon l'invention peuvent être des compositions détergentes telles que des shampooings, des gels-douche et des bains moussants. Dans ce mode de réalisation de l'invention, les compositions comprennent une base lavante, généralement aqueuse.

Le ou les tensioactifs formant la base lavante peuvent être indifféremment choisis, seuls ou en mélanges, au sein des tensioactifs anioniques, amphotères, non ioniques et cationiques tels que définis ci-dessus.

On utilise de préférence un agent tensioactif anionique choisi parmi les alkyl(C₁₂-C₁₄) sulfates de sodium, de triéthanolamine ou d'ammonium, les alkyl (C₁₂-C₁₄)éthersulfates de sodium, de triéthanolamine ou d'ammonium oxyéthylénés à 2,2 moles d'oxyde d'éthylène, le cocoyl iséthionate de sodium et l'alphaoléfine(C₁₄-C₁₆) sulfonate de sodium et leurs mélange avec :
- soit un agent tensioactif amphotère tel que les dérivés d'amine dénommés disodiumcocoamphodipropionate ou sodiumcocoamphopropionate commercialisés notamment par la société RHONE POULENC sous la dénomination commerciale "MIRANOL C2M CONC" en solution aqueuse à 38 % de matière active ou sous la dénomination MIRANOL C32;
- soit un agent tensioactif amphotère de type zwittérionique tel que les alkylbétaïnes en particulier la cocobétaïne commercialisée sous la dénomination "DEHYTON AB 30" en solution aqueuse à 32 % de MA par la société HENKEL.

La quantité et la qualité de la base lavante sont celles suffisantes pour conférer à la composition finale un pouvoir moussant et/ou détergent satisfaisant.

Ainsi, selon l'invention, la base lavante peut représenter de 4 % à 50 % en poids, de préférence de 6 % à 35 % en poids, et encore plus préférentiellement de 8 % à 25 % en poids, du poids total de la composition finale.

L'invention a encore pour objet un procédé de traitement des matières kératiniques telles que la peau ou les cheveux, caractérisé en ce qu'il consiste à appliquer sur les matières kératiniques une composition cosmétique telle que définie précédemment, puis à effectuer éventuellement un rinçage à l'eau.

Ainsi, ce procédé selon l'invention permet le maintien de la coiffure, le traitement, le soin ou le lavage ou le démaquillage de la peau, des cheveux ou de toute autre matière kératinique.

Les compositions de l'invention peuvent également se présenter sous forme d'après-shampooing à rincer ou non, de compositions pour permanente, défrisage, coloration ou décoloration, ou encore sous forme de compositions à rincer, à appliquer avant ou après une coloration, une décoloration, une permanente ou un défrisage ou encore entre les deux étapes d'une permanente ou d'un défrisage.

Lorsque la composition se présente sous la forme d'un après-shampooing éventuellement à rincer, elle contient avantageusement au moins un tensioactif cationique, sa concentrentration généralement comprise entre 0,1 et 10% en poids et de préférence de 0,5 à 5% en poids par rapport au poids total de la composition.

Les compositions de l'invention peuvent encore se présenter sous la forme de compositions lavantes pour la peau, et en particulier sous la forme de solutions ou de gels pour le bain ou la douche ou de produits démaquillants.

Les compositions selon l'invention peuvent également se présenter sous forme de lotions aqueuses ou hydroalcooliques pour le soin de la peau et/ou des cheveux.

Les compositions cosmétiques selon l'invention peuvent se présenter sous forme de gel, de lait, de crème, d'émulsion, de lotion épaissie ou de mousse et être utilisées pour la peau, les ongles, les cils, les lèvres et plus particulièrement les cheveux.

Les compositions peuvent être conditionnées sous diverses formes notamment dans des vaporisateurs, des flacons pompe ou dans des récipients aérosols afin d'assurer une application de la composition sous forme vaporisée ou sous forme de mousse. De telles formes de conditionnement sont indiquées, par exemple, lorsqu'on souhaite obtenir un spray, une laque ou une mousse pour le traitement des cheveux.

Dans tout ce qui suit ou ce qui précède, les pourcentages exprimés sont en poids.

L'invention va être maintenant plus complètement illustrée à l'aide des exemples suivants qui ne sauraient être considérés comme la limitant aux modes de réalisation décrits. Dans les exemples, MA signifie matière active.

Dans les exemples, les noms commerciaux ont les définitions suivantes :

### EXEMPLE 1

On a réalisé un après-shampooing conforme à l'invention de composition suivante :

| | |
|---|---|
| - Inuline (RAFTILINE HP de ORAFTI) | 10,6 g MA |
| - Chlorure de béhényl triméthyl ammonium | 4 g MA |
| - Pectine de citron fortement estérifiée (INSTANT CJ 204 de HERBSTREITH ET FOX) | 1,9 g MA |
| - Amodimethicone (BELSIL ADM 6057 E de WACKER) | 1,7 gMA |
| - Eau qsp | 100 g |

La composition a une texture épaisse et très fondante lors de l'application sur des cheveux humides. Sa rinçabilité est bonne. Les cheveux mouillés ne sont pas chargés et la mise en forme est aisée.

### EXEMPLES 2 A 5

On a réalisé les après-shampooings conformes à l'invention de compositions suivantes :

| | 2 | 3 | 4 |
|---|---|---|---|
| Inuline (RAFTILINE HP de ORAFTI) | 12gMA | 8g | 15g |
| Pectine de citron fortement estérifiée | - | - | 0.7g |
| (INSTANT CJ 204 de HERBSTREITH ET FOX) | | | |
| Gomme de Xanthane | 0.8g | - | - |
| Amidon anionique de pomme de terre oxydé prégélatinisé (PREJEL PA 5 de AVEBE) | - | 5g | - |
| Myristate d'isopropyle | - | 2g | - |
| Amodimethicone (BELSIL ADM 6057 E de WACKER) | - | - | 1.5g |
| Divinyldimethicone / diméthicone réticulé en émulsioncationique (DC2- 1997 de DOW CORNING) | 1.7g | - | - |
| Chlorure de behenyl trimethyl ammonium | 2g | - | - |
| [Chlorure de palmityl amidopropyle triméthyl ammonium | - | 1.8g | - |
| Méthosulfate de dipalmitoyl ethyl hydroxyethyl methyl ammonium / Alcool cétéarylique | - | - | 3.5g |
| Eau qsp | 100g | 100g | 100g |

Les cheveux traités ont les mêmes propriétés que ceux traités avec la composition de l'exemple 1.

## Revendications

1. Composition cosmétique, **caractérisée par le fait qu'**elle comprend, dans un milieu cosmétiquement acceptable, au moins un fructane, au moins un polysaccharide différent du fructane et au moins un agent bénéfique pour les matières kératiniques choisi parmi :
- les cires animales, végétales ou minérales,
- les céramides et les pseudo-céramides,
- les tensioactifs cationiques,
- les huiles minérales, végétales ou animales,
- les esters d'acides gras,
et leurs mélanges.

2. Composition selon la revendication 1, **caractérisée par le fait que** le fructane est choisi parmi les inulines.

3. Composition selon l'une des revendications 1 ou 2, **caractérisée en ce que** les polysaccharides sont choisis parmi les glucanes, les amidons modifiés ou non, l'amylose, l'amylopectine, le glycogène les dextranes, les celluloses et leurs dérivés, les mannanes, les xylanes, les lignines, les arabanes, les galactanes, les galacturonanes, la chitine, les chitosanes, les glucoronoxylanes, les arabinoxylanes, les xyloglucanes, les glucomannanes, les acides pectiques et les pectines, l'acide alginique et les alginates, les arabinogalactanes, les carraghénines, les agars, les glycosaminoglucanes, les gommes arabiques, les gommes Tragacanthe, les gommes Ghatti, les gommes Karaya, les gommes de caroube, les galactomannanes telles que les gommes de guar et leurs dérivés non ioniques (hydroxypropyl guar) et les gommes de xanthane, et leurs mélanges.
(3) les cires animales, végétales ou minérales,
(4) les céramides et les pseudo-céramides,
et leurs mélanges

4. Composition selon la revendication 1, **caractérisée en ce que** les cires animales, végétales ou minérales sont choisis parmi la cire d'abeille, le spermaceti, la cire de lanoline et les dérivés de lanoline ; la cire de Carnauba, de Candelllla, d'Ouricury, du Japon, le beurre de cacao ou les cires de fibres de liège ou de canne à sucre ; les cires de paraffine, de vaseline, de lignite ou les cires microcristallines ou les ozokérites, et leurs mélanges.

5. Composition selon la revendication 1, **caractérisée en ce que** les céramides et les pseudo-céramides sont choisis parmi les céramides des classes I, II, III et V selon la classification de Dawning, et leurs mélanges.

6. Composition selon la revendication 1, **caractérisée en ce que** les tensioactifs cationiques sont choisis parmi les sels d'amines grasses primaires, secondaires ou tertiaires, éventuellement polyoxy-alkylénées ; les sels d'ammonium quaternaire tels que les chlorures ou les bromures de tétraalkylammonium, d'alkylamidoalkyl-trialkylammonium, de trialkylbenzylammonium, de trialkylhydroxyalkyl-ammonium ou d'alkylpyridinium; les dérivés d'imidazoline ; les oxydes d'amines à caractère cationique ; et leurs mélanges.

7. Composition selon la revendication 1, **caractérisée en ce que** les huiles minérales, végétales ou animales sont choisies parmi l'huile de paraffine, l'huile de vaseline, l'huile d'amande douce, l'huile d'avocat, l'huile de ricin, l'huile d'olive, l'huile de jojoba, l'huile de tournesol, l'huile de germes de blé, l'huile de sésame, l'huile d'arachide, l'huile de pépins de raisin, l'huile de soja, l'huile de colza, l'huile de carthame, l'huile de coprah, l'huile de maïs, l'huile de noisette, le beurre de karité, l'huile de palme, l'huile de noyau d'abricot, l'huile de calophyllum, le perhydrosqualène, et leurs mélanges.

8. Composition selon la revendication 1, **caractérisée en ce que** les esters sont choisis parmi les esters d'acides gras comme le myristate d'isopropyle, le palmitate d'isopropyle, le palmitate de 2-éthylhexyle, l'huile de Purcellin (octanoate de stéaryle), l'isononanoate d'isononyle ou d'isostéaryle, le lanolate d'isopropyle, et leurs mélanges.

9. Composition selon l'une quelconque des revendications précédentes, **caractérisée en ce que** ledit fructane est présent à une concentration comprise entre 0,01 % et 20 % en poids par rapport au poids total de la composition, de préférence entre 0,05 % et 10 % en poids.

10. Composition selon l'une quelconque des revendications précédentes, **caractérisée en ce que** ledit polysaccharide est présent à une concentration comprise entre 0,01 % et 20 % en poids par rapport au poids total de la composition, de préférence entre 0,05 % et 10 % en poids.

11. Composition selon l'une quelconque des revendications précédentes, **caractérisée en ce que** l'agent bénéfique est présent à une concentration comprise entre 0,001 % et 20 % en poids par rapport au poids total de la composition, de préférence entre 0,01 % et 10 % en poids.

12. Composition selon l'une quelconque des revendications précédentes, **caractérisée par le fait qu'**elle comprend en outre au moins un agent tensioactif choisi parmi les tensioactifs anioniques, cationiques, non ioniques, amphotères et leurs mélanges.

13. Composition selon la revendication 12, **caractérisée par le fait que** le ou les agents tensioactifs sont présents à une concentration comprise entre 0,1% et 50% en poids, de préférence entre 0,1 % et 40% en poids, et encore plus préférentiellement entre 0,5% et 30% en poids, par rapport au poids total de la composition.

14. Composition selon l'une quelconque des revendications précédentes, **caractérisée par le fait qu'**elle se présente sous forme de shampooing, d'après-shampooing, de composition pour la permanente, le défrisage, la coloration ou la décoloration des cheveux, de composition à rincer à appliquer entre les deux étapes d'une permanente ou d'un défrisage, de composition lavantes pour le corps.

15. Utilisation d'une composition cosmétique telle que définie dans l'une quelconque des revendications précédentes pour le lavage ou pour le soin des matières kératiniques.

16. Procédé de traitement cosmétique des matières kératiniques, telles que les cheveux, **caractérisé en ce qu'**il consiste à appliquer sur lesdites matières une composition cosmétique selon l'une des revendications 1 à 14, puis à effectuer éventuellement un rinçage à l'eau.

## Patentansprüche

1. Kosmetische Zusammensetzung, **dadurch gekennzeichnet, dass** sie in einem kosmetisch unbedenklichen Medium mindestens ein Fructan, mindestens ein von Fructan verschiedenes Polysaccharid und mindestens ein Keratinmaterialien wohltuendes Mittel, ausgewählt aus
- tierischen, pflanzlichen oder mineralischen Wachsen,
- Ceramiden und Pseudoceramiden,
- kationischen Tensiden,
- mineralischen, tierischen oder pflanzlichen Ölen,
- Fettsäureestern,
sowie Mischungen davon, umfasst.

2. Zusammensetzung nach Anspruch 1, **dadurch gekennzeichnet, dass** das Fructan aus Inulinen ausgewählt ist.

3. Zusammensetzung nach einem der Ansprüche 1 oder 2, **dadurch gekennzeichnet, dass** die Polysaccharide aus Glucanen, modifizierten oder unmodifizierten Stärken, Amylose, Amylopektin, Glykogen, Dextranen, Cellulosen und ihren Derivaten, Mannanen, Xylanen, Ligninen, Arabanen, Galactanen, Galacturonanen, Chitin, Chitosanen, Glucoronoxylanen, Arabinoxylanen, Xyloglucanen, Glucomannanen, Pektinsäuren und Pektinen, Alginsäure und Alginaten, Arabinogalactanen, Carrageenen, Agar, Glykosaminoglukanen, Gummi arabicum, Traganth, Ghatti-Gummi, Karayagummi, Johannisbrotkernmehl, Galactomannanen wie Guar und dessen nichtionischen Derivaten (Hydroxypropylguar) und Xanthanen sowie Mischungen davon ausgewählt sind.

4. Zusammensetzung nach Anspruch 1, **dadurch gekennzeichnet, dass** die tierischen, pflanzlichen oder mineralischen Wachse aus Bienenwachs, Walrat, Lanolinwachs und Lanolinderivaten, Carnaubawachs, Candellilawachs, Ouricuriwachs, Japanwachs, Kakaobutter oder Korkfaserwachs oder Zuckerrohrwachs, Paraffinwachsen, Vaseline, Lignitwachs oder mikrokristallinen Wachsen oder Ozokeriten sowie Mischungen davon ausgewählt sind.

5. Zusammensetzung nach Anspruch 1, **dadurch gekennzeichnet, dass** die Ceramide und Pseudoceramide aus den Ceramiden der Klassen I, II, III und V gemäß der Klassifizierung von Dawning sowie Mischungen davon ausgewählt sind.

6. Zusammensetzung nach Anspruch 1, **dadurch gekennzeichnet, dass** die kationischen Tenside aus primären, sekundären oder tertiären, gegebenenfalls polyoxyalkylenierten Fettaminsalzen; quartären Ammoniumsalzen wie Tetraalkylammonium-, Alkylamidoalkyl-trialkylammonium-, Trialkylbenzylammonium-, Trialkylhydroxyalkylammonium- oder Alkylpyridiniuminiumchloriden oder -bromiden; Imidazolinderivaten, Aminoxiden mit kationischem Charakter sowie Mischungen davon ausgewählt sind.

7. Zusammensetzung nach Anspruch 1, **dadurch gekennzeichnet, dass** die mineralischen, pflanzlichen oder tierischen Öle aus Paraffinöl, Vaselineöl, Süßmandelöl, Avocadoöl, Rizinusöl, Olivenöl, Jojobaöl, Sonnenblumenöl, Weizenkeimöl, Sesamöl, Erdnussöl, Traubenkernöl, Sojaöl, Rapsöl, Färberdistelöl, Kokosnussöl, Maisöl, Nussöl, Sheabutter, Palmöl, Aprikosenkernöl, Calophyllumöl, Perhydrosqualen sowie Mischungen davon ausgewählt sind.

8. Zusammensetzung nach Anspruch 1, **dadurch gekennzeichnet, dass** die Ester aus Fettsäureestern wie Isopropylmyristat, Isopropylpalmitat, 2-Ethylhexylpalmitat, Purcellinöl (Stearyloctanoat), Isononyl- oder Isostearylisononanoat, Isopropyllanolat sowie Mischungen davon ausgewählt sind.

9. Zusammensetzung nach einem der vorhergehenden Ansprüche, **dadurch gekennzeichnet, dass** das Fructan in einer Konzentration im Bereich zwischen 0,01 Gew.-% und 20 Gew.-%, bezogen auf das Gesamtgewicht der Zusammensetzung, vorzugsweise zwischen 0,05 Gew.-% und 10 Gew.-%, vorhanden ist.

10. Zusammensetzung nach einem der vorhergehenden Ansprüche, **dadurch gekennzeichnet, dass** das Polysaccharid in einer Konzentration im Bereich zwischen 0,01 Gew.-% und 20 Gew.-%, bezogen auf das Gesamtgewicht der Zusammensetzung, vorzugsweise zwischen 0,05 Gew.-% und 10 Gew.-%, vorhanden ist.

11. Zusammensetzung nach einem der vorhergehenden Ansprüche, **dadurch gekennzeichnet, dass** das wohltuende Mittel in einer Konzentration im Bereich zwischen 0,001 Gew.-% und 20 Gew -%, bezogen auf das Gesamtgewicht der Zusammensetzung, vorzugsweise zwischen 0,01 Gew.-% und 10 Gew.-%, vorhanden ist.

12. Zusammensetzung nach einem der vorhergehenden Ansprüche, **dadurch gekennzeichnet, dass** sie darüber hinaus mindestens ein Tensid, ausgewählt aus anionischen, kationischen, nichtionischen oder amphoteren Tensiden und Mischungen davon, umfasst.

13. Zusammensetzung nach Anspruch 12, **dadurch gekennzeichnet, dass** das oder die Tenside in einer Konzentration im Bereich zwischen 0,1 Gew.-% und 50 Gew.-%, vorzugsweise zwischen 0,1 und 40 Gew.-% und noch mehr bevorzugt zwischen 0,5 und 30 Gew.-%, bezogen auf das Gesamtgewicht der Zusammensetzung, vorhanden sind.

14. Zusammensetzung nach einem der vorhergehenden Ansprüche, **dadurch gekennzeichnet, dass** sie in Form eines Shampoos, einer Haarspülung, einer Zusammensetzung für eine Dauerwelle, eine Haarglättung, eine Färbung oder Bleichung der Haare, einer Spülungszusammensetzung zur Anwendung zwischen den beiden Schritten einer Dauerwelle oder Haarglättung, und als Waschzusammensetzung für den Körper vorliegt.

15. Verwendung einer kosmetischen Zusammensetzung gemäß einem der vorhergehenden Ansprüche zum Waschen oder Pflegen von Keratinmaterial.

16. Verfahren zur kosmetischen Behandlung von Keratinmaterial, wie den Haaren, **dadurch gekennzeichnet, dass** es darin besteht, eine kosmetische Zusammensetzung nach einem der Ansprüche 1 bis 14 auf diese Materialien aufzubringen und dann gegebenenfalls mit Wasser zu spülen.

## Claims

1. Cosmetic composition, **characterized in that** it comprises, in a cosmetically acceptable medium, at least one fructan, at least one polysaccharide other than the fructan and at least one beneficial agent for keratinous substances chosen from:
- animal, vegetable or mineral waxes,
- ceramides and pseudoceramides,
- cationic surfactants,
- mineral, vegetable or animal oils,
- esters of fatty acids,
and their mixtures.

2. Composition according to Claim 1, **characterized in that** the fructan is chosen from inulins.

3. Composition according to either of Claims 1 and 2, **characterized in that** the polysaccharides are chosen from glucans, modified or unmodified starches, amylose, amylopectin, glycogen, dextrans, celluloses and their derivatives, mannans, xylans, lignins, arabans, galactans, galacturonans, chitin, chitosans, glucoronoxylans, arabinoxylans, xyloglucans, glucomannans, pectic acids and pectins, alginic acid and alginates, arabinogalactans, carrageenins, agars, glycosaminoglucans, gums arabic, gums tragacanth, ghatti gums, karaya gums, locust bean gums, galactomannans, such as guar gums and their nonionic derivatives (hydroxypropyl guar), and xanthan gums, and their mixtures.

4. Composition according to Claim 1, **characterized in that** the animal, vegetable or mineral waxes are chosen from beeswax, spermaceti, lanolin wax and lanolin derivatives; carnauba, candelilla, ouricury or Japan wax, cocoa butter or cork fiber or sugarcane waxes; paraffin, petrolatum or lignite waxes or microcrystalline waxes or ozokerites, and their mixtures.

5. Composition according to Claim 1, **characterized in that** the ceramides and pseudoceramides are chosen from the ceramides of the classes I, II, III and V according to the Dawning classification, and their mixtures.

6. Composition according to Claim 1, **characterized in that** the cationic surfactants are chosen from salts of primary, secondary or tertiary fatty amines which are optionally polyoxyalkylenated; quaternary ammonium salts, such as tetraalkylammonium, alkylamidoalkyltrialkylammonium, trialkylbenzyl-ammonium, trialkylhydroxyalkylammonium or alkylpyridinium chlorides or bromides; imidazoline derivatives; amine oxides with a cationic nature; and their mixtures.

7. Composition according to Claim 1, **characterized in that** the mineral, vegetable or animal oils are chosen from liquid paraffin, liquid petrolatum, sweet almond oil, avocado oil, castor oil, olive oil, jojoba oil, sunflower oil, wheat germ oil, sesame oil, groundnut oil, grape seed oil, soybean oil, rapeseed oil, safflower oil, coconut oil, corn oil, hazelnut oil, karite butter, palm oil, apricot kernel oil, calophyllum oil, perhydrosqualene, and their mixtures.

8. Composition according to Claim 1, **characterized in that** the esters are chosen from esters of fatty acids, such as isopropyl myristate, isopropyl palmitate, 2-ethylhexyl palmitate, purcellin oil (stearyl octanoate), isononyl isononanoate, isostearyl isononanoate, isopropyl lanolate, and their mixtures.

9. Composition according to any one of the preceding claims, **characterized in that** said fructan is present at a concentration of between 0.01% and 20% by weight with respect to the total weight of the composition, preferably between 0.05% and 10% by weight.

10. Composition according to any one of the preceding claims, **characterized in that** said polysaccharide is present at a concentration of between 0.01% and 20% by weight with respect to the total weight of the composition, preferably between 0.05% and 10% by weight.

11. Composition according to any one of the preceding claims, **characterized in that** the beneficial agent is present at a concentration of between 0.001% and 20% by weight with respect to the total weight of the composition, preferably between 0.01% and 10% by weight.

12. Composition according to any one of the preceding claims, **characterized in that** it additionally comprises at least one surface-active agent chosen from anionic, cationic, nonionic or amphoteric surfactants and their mixtures.

13. Composition according to Claim 12, **characterized in that** the surface-active agent or agents are present at a concentration of between 0.1% and 50% by weight, preferably between 0.1% and 40% by weight and more preferably still between 0.5% and 30% by weight, with respect to the total weight of the composition.

14. Composition according to any one of the preceding claims, **characterized in that** it is provided in the form of a shampoo, conditioner, composition for perming, straightening, dyeing or bleaching the hair, rinse-out composition to be applied between the two stages of a permanent wave or hair straightening, or washing composition for the body.

15. Use of a cosmetic composition as defined in any one of the preceding claims for washing or caring for keratinous substances.

16. Process for the cosmetic treatment of keratinous substances, such as the hair, **characterized in that** it consists in applying, to said substances, a cosmetic composition according to one of Claims 1 to 14 and in then optionally rinsing with water.
